# EUROPÄISCHE PATENTANMELDUNG

(11) **EP 0 790 230 A1**
(43) Veröffentlichungstag der Anmeldung: **20.08.1997**
(21) Anmeldenummer: 97101734.8
(22) Anmeldetag: 04.02.1997
(51) Int. Cl.: C07C 67/08, C07C 67/54, C07C 69/54

(54) **Verfahren zur kontinuierlichen Herstellung von Alkylestern der (Meth)acrylsäure**

(30) Priorität: 06.02.1996 DE 19604267
(71) Anmelder: BASF AKTIENGESELLSCHAFT, 67056 Ludwigshafen (DE)
(72) Erfinder: Iffland, Gabriele, 67071 Ludwigshafen (DE); Dams, Albrecht, Dr., 67157 Wachenheim, (DE); Weck, Alexander, 77830 Bühlertal (DE); Aichinger, Heinrich, Dr., 68199 Mannheim (DE); Herbst, Holger, Dr., 67227 Frankenthal (DE); Nestler, Gerhard, Dr., 67061 Ludwigshafen (DE); Exner, Herbert, Dr., 67165 Waldsee (DE); Schmidt, Willi, 67069 Ludwigshafen (DE); Geisendörfer, Matthias, Dr., 67435 Neustadt (DE); Dockner, Toni, Dr., 67149 Meckenheim (DE)
(74) Vertreter: Isenbruck, Günter, Dr.

(57) **Zusammenfassung**

Bei dem Verfahren zur kontinuierlichen Herstellung von Alkylestern der (Meth)acrylsäure durch Umsetzung von (Meth)acrylsäure mit 1 bis 8 C-Atome aufweisenden einwertigen Alkanolen in Gegenwart eines sauren Veresterungkatalysators in einer Reaktionszone wird aus der Reaktionszone ein Produktgemisch ausgetragen, das den gebildeten Alkylester der (Meth)acrylsäure, den Katalysator und die im Verlauf der Veresterung gebildeten höher als der Alkylester der (Meth)acrylsäure siedenden Nebenprodukte enthält, und der Alkylester der (Meth)acrylsäure wird in einer Trennzone destillativ abgetrennt. Das aus der Reaktionszone ausgetragene Produktgemisch wird einer Rektifikationseinheit (I) zugeführt, in dieser wird das ausgetragene Produktgemisch rektifikativ in wenigstens ein den Alkylester der (Meth)acrylsäure enthaltendes Produkt (I) und ein den Katalysator enthaltendes Produkt (II) aufgetrennt, das Produkt (I) wird einer weiteren Rektifikationseinheit (II) zugeführt und in dieser wird der Alkylester der (Meth)acrylsäure rektifikativ abgetrennt.

## Beschreibung

Die Erfindung betrifft ein Verfahren zur kontinuierlichen Herstellung von Alkylestern der (Meth)acrylsäure durch Umsetzung von (Meth)acrylsäure mit 1 bis 8 C-Atome aufweisenden einwertigen Alkanolen in Gegenwart eines sauren Veresterungkatalysators.

Der Begriff (Meth)acrylsäure bezeichnet dabei in bekannter Weise Acryl- oder Methacrylsäure. Bei Veresterungen von Alkanol mit organischer Säure laufen generell typische Gleichgewichtsreaktionen ab, die durch starke Säuren katalysiert werden und die als typische Kondensationsreaktionen zur Abspaltung von Wasser führen. Üblicherweise wird durch Entfernung des Wassers aus dem Reaktionsgemisch das Veresterungsgleichgewicht in die gewünschte Richtung verschoben. Die Abtrennung des Wassers kann destillativ als Bestandteil eines Azeotrops erfolgen, das auch den Zielester umfaßt. Mit der kontinuierlichen Abtrennung des Reaktionswassers aus dem Reaktionsgemisch geht gleichzeitig die Abtrennung des Zielesters vom Reaktionsgemisch einher. In der Regel erfolgt die Veresterungsreaktion so, daß zwar das Wasser kontinuierlich aus dem Reaktionsgemisch entfernt wird, die Hauptmenge des gebildeten Zielesters aber im Reaktionsgemisch verbleibt.

Beispiele für Veresterungen dieser Art bilden diejenigen, bei denen das Reaktionswasser durch Zusatz eines organischen Lösungsmittels als azeotropem Schlepper destillativ abgetrennt wird. Als ein solches azeotropes Schleppmittel kann aber auch im Überschuß eingesetztes (Ausgangs)alkanol dienen. Eine andere Variante besteht darin, daß das Wasser als Bestandteil eines Azeotrops aus Zielester/Alkanol/Wasser destillativ abgetrennt wird, das zu mehr als 95 Gew.-% aus Wasser besteht.

Die bei solchen Veresterungen entstehenden Produktgemische enthalten im wesentlichen überschüssiges Alkanol, überschüssige (Meth)acrylsäure, sauren Veresterungskatalysator sowie Polymerisationsinhibitoren, da (Meth)acrylsäure und ihre Ester zur Polymerisation neigen. Ferner können gegebenenfalls azeotrope Schleppmittel oder organische Losungsmittel, Restmengen an Wasser und schwersiedende Nebenprodukte (Michael-Addukte) enthalten sein. Aus diesen Produktgemischen muß dann der Zielester abgetrennt werden. Gemäß Ullmann's Encyclopedia of Industrial Chemistry, 5th edition, Vol. A1, VCH Weinheim, Seite 168/169 erfolgt diese Abtrennung in der Regel so, daß das Produktgemisch zunächst mit Wasser gewaschen wird. Der saure Verestetungskatalysator und die überschüssige (Ausgangs)säure gehen aus der organischen Produktgemischphase in die Wasserphase und werden so aus dem Produktgemisch abgetrennt. Durch Nachwaschen mit wäßriger Alkalilauge wird diese Abtrennung normalerweise vervollständigt.

Aus der verbleibenden organischen Phase wird anschließend in einer ersten Rektifikationskolonne regelmäßig zunächst das verbliebene Alkanol und danach in einer weiteren Rektifikationskolonne der Zielester jeweils über Kopf abgetrennt.

Die Nachteile einer solchen Aufarbeitungsweise liegen insbesondere im Anfall von großen Mengen stark belasteten Abwassers. Außerdem ist die in der wäßrigen Alkalilauge gelöste Säure und das darin gelöste Alkanol in der Regel nicht direkt und technisch aufwendig in die Veresterung zurückführbar, was Eduktverluste bedingt.

Ein wasserfreies Aufarbeitungsverfahren ist beispielsweise aus der DE-C 25 48 561 für die Herstellung von 2-Ethylhexylacrylat bekannt. Bei der dort beschriebenen Aufarbeitungsweise werden überschüssiges Alkanol und überschüssige Säure über Kopf vom Produktgemisch destillativ abgetrennt. In einer nachfolgenden Destillationskolonne wird dann aus dem Sumpfprodukt der vorhergehenden Destillationskolonne der Zielester destillativ abgetrennt. Das Sumpfprodukt, aus dem die destillative Abtrennung des Zielesters erfolgt, enthält allerdings noch den sauren Katalysator der eigentlichen Veresterungsreaktion. Außerdem erfordert die destillative Abtrennung des Zielesters erhöhte Temperaturen auch bei reduziertem Druck. Dies führt dazu, daß bei der destillativen Abtrennung eine Rückspaltung der im Rahmen der eigentlichen Veresterung als Nebenprodukte gebildeten Michael-Addukte auftritt. Im Rahmen der Rückspaltung entstehen dann leichter als der Zielester siedendes Alkanol und Säure sowie durch Wasser-Elimination aus dem Ausgangsalkohol gegebenenfalls leichtsiedende Olefine, die dann als Verunreinigungen im destillativ abgetrennten Zielester enthalten sind. Die Reinheit eines so gewonnenen Zielesters ist unbefriedigend.

Die GB-B 1 017 522 offenbart ein Verfahren zur Herstellung von n-Butylacrylat. Als Veresterungsbedingungen wird hier ein bestimmtes molares Verhältnis von (Ausgangs)alkanol zu (Ausgangs)säure beschrieben sowie ein auf die Gesamtmasse der Reaktanden bezogener Gehalt von 0,5 bis 5 Gew.-% an katalytisch wirksamer Säure. Nachteilig an dieser Verfahrensweise ist der erforderliche erhöhte Überschuß an Alkanol, der die Bildung an unerwünschtem Dialkylether fördert, sowie die unter vorgenannten Bedingungen nicht voll befriedigende Ausbeute an n-Butylacrylat bezogen auf die eingesetzte Menge an Acrylsäure.

Aus der DE-C 25 52 987 ist ein Verfahren zu kontinuierlichen Herstellung von Alkylestern der Acrylsäure durch Umsetzung von Acrylsäure und 1 bis 4 C-Atome aufweisenden einwertigen Alkanolen in homogener, flüssiger, an Lösungsmittel freier Phase im Molverhältnis von 1(Alkanol):1(Acrylsäure) bis 2(Alkanol):1(Acrylsäure) bei erhöhter Temperatur und in Gegenwart von Schwefelsäure oder organischer Sulfonsäure als Katalysator bekannt, bei dem man die Acrylsäure, das Alkanol und den Säurekatalysator kontinuierlich einer Reaktionszone zuführt, während einer Verweilzeit von einigen Stunden den gebildeten Acrylsäurealkylester als Bestandteil wenigstens eines neben dem Acrylsäurealkylester als weiteren Bestandteil aus Wasser oder Wasser und Alkanol bestehenden wäßrigen Azeotrops über Kopf einer der Reaktionszone aufgesetzten Rektifikationskolonne rektifikativ abtrennt, das anfallende Destillat I in eine den gebildeten Acrylsäureester enthaltende organische und in eine wäßrige Phase auftrennt, einen Teil der organischen Phase zum Zwecke der Erzeugung einer erhöhten Trennwirkung sowie gegebenenfalls einen Teil der wäßrigen Phase zur Aufrechterhaltung der Zusammensetzung des wäßrigen Azeotrops über Kopf der Rektifikationszone zurückführt, aus der überschüssigen organischen Phase den Alkylester in an sich bekannter Weise abtrennt und einen Teil des Reaktionsgemisches aus der Reaktionszone austrägt, von Hochsiedern destillativ befreit und das dabei anfallende Destillat in die Reaktionszone zurückführt.

Vorrangige Zielsetzung ist hier die Vermeidung unerwünschter Etherbildung aus (Ausgangs)alkanol. Nachteilig an dieser Verfahrensweise ist jedoch, daß trotz destillativer Behandlung des Austrags aus dem Reaktionsgemisch und Rückführung des dabei anfallenden Destillats in die Reaktionszone die Ausbeute an Alkylacrylat, bezogen auf eingesetzte Acrylsäure, nicht zu befriedigen vermag. Auch die erreichte Verringetung der Dialkylether-Nebenproduktbildung kann nicht voll befriedigen. Ferner vermag auch die gemäß den Ausführungsbeispielen erforderliche Verweilzeit nicht zu befriedigen. Dies gilt auch für die Raum-Zeit-Ausbeute. Es wird angenommen, daß dies auf der geringen Konzentration an saurem Veresterungskatalysator beruht.

Der Erfindung liegt die Aufgabe zugrunde, ein Verfahren zur kontinuierlichen Herstellung von Alkylestern der (Meth)acrylsäure zu schaffen, das neben einer optimierten Ausbeute mildere Reaktionsbedingungen und damit neben stark verminderter Etherbildung weniger Hochsiederbildung, eine hohe Raum-Zeit-Ausbeute, eine erhöhte Flexibilität des Betriebs der Anlage sowie niedrige Investitionskosten aufgrund einer minimierten Apparatezahl ermöglicht.

Die Lösung geht aus von dem bekannten Verfahren zur kontinuierlichen Herstellung von Alkylestern der (Meth)acrylsäure durch Umsetzung von (Meth)acrylsäure mit 1 bis 8 C-Atome aufweisenden einwertigen Alkanolen in Gegenwart eines sauren Veresterungkalalysators in einer Reaktionszone, bei dem aus der Reaktionszone ein Produktgemisch ausgetragen wird, das den gebildeten Alkylester der (Meth)acrylsäure, den Katalysator und die im Verlauf der Veresterung gebildeten höher als der Alkylester der (Meth)acrylsäure siedenden Nebenprodukte enthält und aus dem der Alkylester der (Meth)acrylsäure in einer Trennzone destillativ abgetrennt wird.

Das erfindungsgemäße Verfahren ist dann dadurch gekennzeichnet, daß man das aus der Reaktionszone ausgetragene Produktgemisch einer Rektifikationseinheit I zuführt, in dieser das ausgetragene Produktgemisch rektifikativ in wenigstens ein den Alkylester der (Meth)acrylsäure enthaltendes Produkt I und ein den Katalysator enthaltendes Produkt II auftrennt, das Produkt I einer weiteren Rektifikationseinheit II zuführt und in dieser den Alkylester der (Meth)acrylsäure rektifikativ abtrennt.

Der Begriff Rektifikationseinheit ist hier wie auch im folgenden als allgemeine Bezeichnung für Apparate zu verstehen, in denen durch Wärmezufuhr Dämpfe erzeugt werden, die aufsteigen und in Kontakt mit abströmender flüssiger Phase stehen. Hierzu sind auch einfache Destillationskolonnen zu rechnen. In der Regel handelt es sich hierbei jedoch um Rektifikationskolonnen, in denen Einbauten für den intensiven Kontakt zwischen Flüssigkeit und Dampf enthalten sind. Derartige Einbauten sind Böden, wie Glockenboden, Lochböden, insbesondere Dual-Flow-Böden, Schüttungen, Packungen oder dergleichen. Zur Vereinfachung des Verständnisses der Zusammenhänge sind die verschiedenen Rektifikationseinheiten mit römischen Ziffern bezeichnet. Auch die verschiedenen, im einzelnen beschriebenen Produkte tragen eine derartige Bezeichnung.

Erfindungsgemäß wird also in einer ersten Rektifikationseinheit I das aus der Reaktionszone ausgetragene Produktgemisch rektifikativ in wenigstens ein den zu gewinnenden Alkylester der (Meth)acrylsäure enthaltendes Produkt I aufgetrennt, das dem oberen Teil der Rektifikationseinheit I entnommen werden kann, sowie in ein im unteren Teil der Rektifikationseinheit I anfallendes Produkt II, das den Katalysator enthält. Das den Alkylester der (Meth)acrylsäure enthaltende und im oberen Teil der Rektifikationseinheit I entnommene Produkt I wird einer weiteren Rektifikationseinheit II zugeführt und in dieser wird dann der zu gewinnende reine Alkylester der (Meth)acrylsäure rektifikativ abgetrennt.

Gemäß einer vorteilhaften Ausbildung der Erfindung findet die Veresterung in der Reaktionszone in homogener, flüssiger, an Lösungsmittel freier Phase bei erhöhter Temperatur durch Umsetzung von (Meth)acrylsäure und den einwertigen Alkanolen in einem Molverhältnis von 1:0,75 bis 1:2, vorzugsweise 1:0,9 bis 1:1,1, besonders bevorzugt im Verhältnis 1:1 statt. Dabei führt man der Reaktionszone die (Meth)acrylsäure, das Alkanol und den Katalysator zu und trennt während einer Verweilzeit das gebildete Wasser als Bestandteil eines Alkanol umfassenden Gemischs über Kopf einer der Reaktionszone aufgesetzten Rektifikationseinheit III rektifikativ ab. Das dabei anfallende Destillat wird in eine Ausgangsalkanol enthaltende organische und in eine Wasser enthaltende wäßrige Phase aufgetrennt. Die organische Phase und gegebenenfalls wäßrige Phase wird in die Rektifikationseinheit III zurückgeführt. Das so weitgehend von Wasser befreite Reaktionsgemisch wird aus der Reaktionszone ausgetragen und einer Trennzone zugeführt.

Die Reaktionszone besteht aus einem oder mehreren Reaktionsbereichen. Bei einer Ausführungsform der Erfindung mit mehreren Reaktionsbereichen ist es vorteilhaft, diese zu kaskadieren. Der flüssige Austragsstrom eines Reaktionsbereichs bildet dabei den Zulauf des nachfolgenden Reaktionsbereichs. Dies kann mit Hilfe eines Überlaufs geschehen. Für den Fall, daß es sich bei den einzelnen Reaktionsbereichen um voneinander getrennte Apparate handelt, ist deren Anzahl unter Berücksichtigung der Investitionskosten ≥ 2 und ≤ 4. Wird mehr als ein Reaktionsbereich innerhalb ein und desselben Reaktors geschaffen (z.B. durch den Einsatz von Trennblechen), so kann die Anzahl der Reaktionsbereiche auch größer 4 sein. Im Falle von mehreren Reaktionsbereichen werden die Brüden der Reaktionsbereiche einer gemeinsamen Rektifikationskolonne zugeführt, deren flüssiger Ablauf vorteihafterweise in den ersten Reaktionsbereich gelangt.

Wird bei diesem Verfahren ein Alkanol eingesetzt, das 4 - 8 C-Atome aufweist, beträgt die Temperatur im ersten Reaktionsbereich in der Regel 70 - 150°C, vorzugsweise 80 - 130°C, und im letzten Bereich 100 - 160°C, vorzugsweise 110 - 130°C. Die Reaktionstemperatur wird vorzugsweise so eingestellt, daß sie längs der Kaskade ansteigt. Der Druck in allen Reaktionsbereichen beträgt von 100 mbar bis Atmosphärendruck, vorzugsweise 200 mbar - 700 mbar. Vorteilhafterweise ist der Druck in allen Reaktionsbereichen gleich. Die Gesamtverweilzeit der Reaktanden im Reaktionsbereich beträgt 0,5 - 10 h, vorzugsweise 1 - 7 h, besonders bevorzugt 2 - 5 h.

Bevorzugt wird als saurer Veresterungskatalysator p-Toluolsulfonsäure eingesetzt. Ihr Gehalt in der Reaktionszone bezogen auf das darin enthaltene Reaktionsgemisch beträgt 0,1 - 10 Gew.-%, bevorzugt 0,1 - 6 Gew.-%. Saure Veresterungskatalysatoren wie Schwefelsäure und/oder andere organische Sulfonsäuren sind ebenfalls einsetzbar.

In der Regel werden sowohl die (Meth)acrylsäure als auch der Katalysator der Reaktionszone direkt zugeführt. Das zu veresternde Alkanol wird vorzugsweise der Reaktionszone über die auf diese aufgesetzte Rektifikationseinheit III zugeführt. Diese Rektifikationseinheit III kann aus einer Rektifikationskolonne bekannter Bauart beispielsweise mit Glockenböden oder Siebböden bestehen. Die Reaktionsbereiche können vorteilhafterweise aus Reaktoren mit Natur- oder Zwangsumlaufverdampfern bestehen.

Je nachdem, welches Alkanol verestert werden soll, sind in Einzelheiten voneinander abweichende Verfahrensweisen sinnvoll und zweckmäßig. Bei der Umsetzung von niederen Alkanolen sind die Siedepunkte des Esters und der (Meth)acrylsäure so nahe beieinander, daß diese in der Rektifikationseinheit II nicht mehr wirtschaftlich destillativ abgetrennt werden können, dies gilt im besonderen für n-Butylacrylat. Aus diesem Grund wird bereits in der Rektifikationseinheit I Wasser zugeführt, um die (Meth)acrylsäure im Sumpf zu halten. Die hierdurch sich ergebenden Verfahrensschritte werden anhand der Veresterung von n-Butanol mit (Meth)acrylsäure beschrieben.

Die Veresterung von Alkanolen mit mehr als 4 C-Atomen, insbesondere mit 8 C-Atomen, bei denen die Siedepunkte des Esters und der (Meth)acrylsäure weiter auseinander liegen und daher in der Rektifikationseinheit I keine Wasserzuführung erfolgen muß, werden anhand des Beispiels der Veresterung von 2-Ethylhexanol näher beschrieben.

Zunächst also wird im folgenden die Veresterung von Alkanolen mit 1 - 4 C-Atomen mit (Meth)acrylsäure am Beispiel von n-Butanol als Alkanol beschrieben. Dabei wird die am Kopf der Rektifikationseinheit III anfallende wäßrige Phase ausgeschleust, bevorzugt vollständig. Das aus der Reaktionszone ausgetragene Produktgemisch wird der Rektifikationseinheit I unter Zusatz von Wasser zugeführt. Das in diese Rektifikationseinheit I unter Zusatz von Wasser eingeführte Produktgemisch wird in dieser in ein den Katalysator und die verbliebene (Meth)acrylsäure enthaltendes Produkt II und in ein den n-Butylester der (Meth)acrylsäure, verbliebenes n-Butanol und Wasser enthaltendes Produkt aufgetrennt. Das das Wasser enthaltende Produkt kann dann in eine den n-Butylester der (Meth)acrylsäure und n-Butanol enthaltende organische Phase und in eine wäßrige Phase aufgetrennt werden. Vorteilhafterweise wird die wäßrige Phase dann teilweise in die Rektifikationseinheit I zurückgeführt. Diese Rektifikationseinheit ist hier als Rektifikationskolonne der zuvor beschriebenen Bauart ausgebildet. Das aus der Reaktionszone ausgetragene Produktgemisch wird dem unteren Teil der Rektifikationskolonne I zugeführt und der Wasserzusatz wird bevorzugt in den oberen Teil der Rektifikationskolonne I eingeführt. In der Rektifikationseinheit I werden eine flüssige wäßrige und eine flüssige organische Phase erzeugt.

Vorteilhafterweise wird ein Teil der den n-Butylester der (Meth)acrylsäure und n-Butanol enthaltenden anfallenden organischen Phase in den oberen Teil der Rektifikationskolonne I zurückgeführt. Das in der Rektifikationseinheit I anfallende, den Katalysator und die verbliebene (Meth)acrylsäure enthaltende Produkt II kann dabei im wesentlichen vollständig in die Reaktionszone, und zwar vorzugsweise in den ersten Rekationsbereich, entweder direkt und/oder über die Rektifikationseinheit III zurückgeführt werden. Bei dieser Verfahrensweise kann ein Teil des in der Rektifikationseinheit I anfallenden Produktes II ausgetragen und einer weiteren Destillationseinheit IV zugeführt werden. In dieser kann das Produkt in ein n-Butanol, (Meth)acrylsäure und den n-Butylester der (Meth)acrylsäure enthaltendes Produkt III und ein den Katalysator sowie höher als den n-Butylester der (Meth)acrylsäure siedende Komponenten enthaltendes Produkt IV aufgetrennt werden. Das Produkt III, das n-Butanol, (Meth)acrylsäure und den n-Butylester der (Meth)acrylsäure enthält, kann dann in die Rektifikationseinheit I und/oder in die Reaktionszone zurückgeführt werden.

Die organische Phase des Produkts I wird vorteilhafterweise einer Rektifikationseinheit II zugeführt und in dieser aufgetrennt in a) ein Produkt V, das verbliebenes n-Butanol und leichter als n-Butyl(meth)acrylat siedende Komponenten enthält, b) den Zielester n-Butyl(meth)acrylat und c) ein Produkt VI, das schwerer als n-Butyl(meth)acrylat siedende Bestandteile aufweist. Das Produkt V wird in die Reaktionszone zurückgeleitet, vorzugweise über die Rektifikationseinheit III. Das Produkt VI wird in die Rektifikationseinheit I zurückgeführt. Die Rektifikationseinheit II kann als Rektifikationskolonne II bereits beschriebener Bauart ausgebildet sein. Das vorgenannte Produkt V wird im oberen Teil der Rektifikationskolonne II, das Produkt VI wird im Sumpf der Rektifikationskolonne II und das n-Butyl(meth)acrylat wird dampfförmig mit Hilfe eines Seitenabzugs im unteren Teil der Rektifikationskolonne II abgetrennt. Das dampfförmige Zielprodukt n-Butyl(meth)acrylat wird dann kondensiert und kann anschließend mit einem Lagerstabilisator versetzt werden.

Im folgenden soll nun die Veresterung mit Alkanolen von 5 - 8 C-Atomen anhand des Beispiels der Veresterung mit 2-Ethylhexanol beschrieben werden. Hier wird das aus der Reaktionszone ausgetragene Produktgemisch der Rektifikationseinheit I zugeführt. Von der am Kopf der Rektifikationseinheit III anfallenden wäßrigen Phase wird ein Teil in die Rektifikationseinheit III zurückgeführt. Das in die Rektifikationseinheit I geführte Produktgemisch wird in dieser in ein den 2-Ethylhexylester der (Meth)acrylsäure, verbliebenes 2-Ethylhexanol und verbliebene (Meth)acrylsäure enthaltendes Produkt VII und in ein den Katalysator und schwerer als den 2-Ethylhexylester der (Meth)acrylsäure siedende Komponenten enthaltendes Produkt VIII aufgetrennt. Dabei wird zweckmäßigerweise als Rektifikationseinheit I wieder eine Rektifikationskolonne I eingesetzt. Dieser wird das aus der Reaktionszone ausgetragene Produktgemisch im unteren Teil zugeführt. Das Produkt VIII wird aus dem Sumpf dieser Rektifikationskolonne und das Produkt VII an deren Kopf gewonnen. Ein Teil des Produkts VIII wird zweckmäßigerweise in die Reaktionszone, vorzugsweise in den ersten Reaktionsbereich entweder direkt und/oder über die Rektifikationseinheit III zurückgeführt. Vorteilhafterweise wird ein Teil des Produkts VIII ausgetragen und einer Destillationseinheit IV zugeführt und in dieser aufgetrennt in ein 2-Ethylhexanol, (Meth)acrylsäure und 2-Ethylhexylester der (Meth)acrylsäure enthaltendes Produkt IX und ein den sauren Veresterungskatalysator sowie höher als der 2-Ethylhexylester der (Meth)acrylsäure siedende Komponenten enthaltendes Produkt X.

Das Produkt IX kann dann in die Rektifikationseinheit I und/oder in die Reaktionszone rückgeführt werden. Aus dem Produkt VIII und/oder dem Produkt X können durch Extraktion mit Wasser saurer Veresterungskatalysator teilweise oder vollständig abgetrennt und die anfallende wäßrige Phase teilweise oder vollständig in die Reaktionszone zurückgeführt werden. Zu dieser Extraktion kann ein Teil der in der Rektifikationseinheit III anfallenden wäßrigen Phase verwendet werden. Das der Rektifikationseinheit I entnommene Produkt VII kann der Rektifikationseinheit II zugeführt und in dieser aufgetrennt werden in a) ein Produkt XI, das verbliebenes 2-Ethylhexanol, (Meth)acrylsäure und leichter als 2-Ethylhexyl(meth)acrylat siedende Komponenten enthält, die b) in den Zielester 2-Ethylhexyl(meth)acrylat und c) ein Produkt XII, das schwerer als 2-Ethylhexyl(meth)acrylat enthaltende Bestandteile aufweist. Das Produkt XI kann dann in die Reaktionszone zurückgeführt werden, vorzugsweise über die Rektifikationseinheit III, das Produkt XII kann in die Rektifikationseinheit I zurückgeführt werden. Die Rektifikationseinheit II ist zweckmäßigerweise als Rektifikationskolonne ausgebildet. Hier kann das Produkt XI im oberen Teil, das Produkt XII ihrem Sumpf und das 2-Ethylhexyl(meth)acrylat als dampfförmiger Seitenabzug im unteren Teil abgetrennt werden.

Weitere Einzelheiten und Vorteile der Erfindung können den anhand der Zeichnung beschriebenen Ausführungsbeispielen entnommen werden. Dabei zeigen:
- Fig. 1: das Verfahrensschema einer Anlage zur Herstellung von n-Butylacrylat
- Fig. 2: das Verfahrensschema einer Anlage zur Herstellung von 2-Ethylhexylacrylat.

Gleiche oder gleichwirkende Apparateteile sind mit gleichen Bezugszeichen versehen. Die Rektifikationskolonnen sind mit römischen Bezugszeichen versehen. Der Übersichtlichkeit wegen sind außerdem die allgemein mit römischen Ziffern versehenen Produktbezeichnungen in den speziellen Ausführungsbeispielen konkret eingefügt.

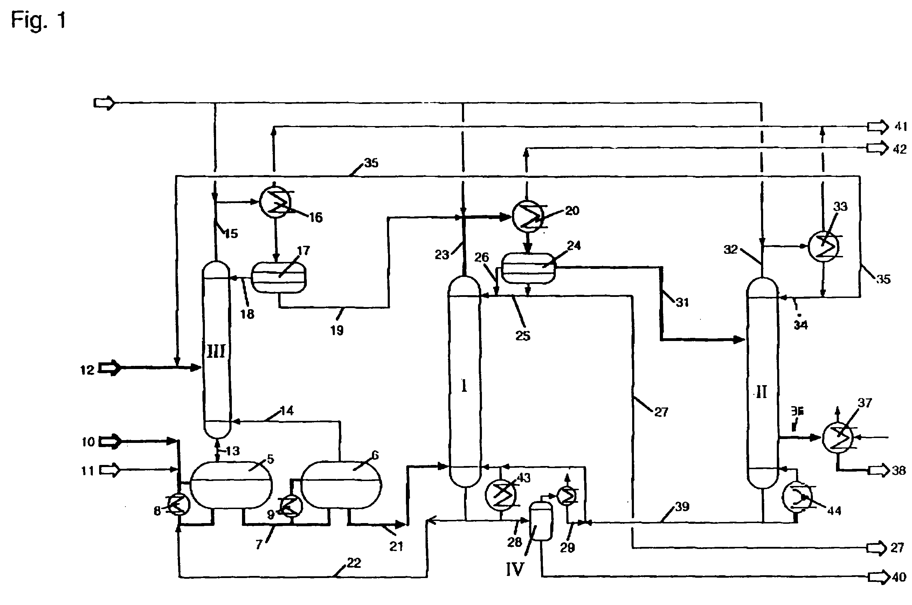

Die in Fig. 1 dargestellte Anlage zur Durchführung des erfindungsgemäßen Verfahrens für die Herstellung von n-Butylacrylat hat drei Rektifikationskolonnen I, II, III, und eine Destillationseinheit IV. Sie ist außerdem mit zwei Veresterungsreaktoren 5 und 6 versehen, die über eine Leitung 7 hintereinander geschaltet sind und so eine Reaktionskaskade bilden. An die Reaktoren 5 und 6 sind Umlaufverdampfer 8 und 9 angeschlossen. Über die Leitung 10 wurden 4 mol/h Acrylsäure dem ersten Reaktor 5 und über die auf den ersten Reaktor 5 aufgesetzte Kolonne III 4 mol/h n-Butanol zugeführt, das in diese Kolonne III durch die Leitung 12 eingeleitet wurde. (Der Einfachheit halber steht im Folgenden Butanol für n-Butanol.) In den ersten Reaktor 5 wurde außerdem als Katalysator wäßrige p-Toluolsulfonsäure über die Leitung 11 eingeführt in einer Menge von 1,5 Gew.-% bezogen auf die eingesetzten Edukte. Die Umsetzung im ersten Reaktor 5 wurde bei einer Temperatur von 100°C, im nachgeschalteten zweiten Reaktor 6 bei einer Temperatur von 105°C bei einem Systemdruck von 380 mbar und einer Verweilzeit in der Reaktionszone von ca. 3 h durchgeführt.

Die aus den Reaktoren 5 und 6 aufsteigendem Dämpfe wurden durch Leitungen 13 und 14 in eine mit 25 Glockenböden ausgestattete und bei 360 mbar Kopfdruck betriebene Kolonne III als erste Rektifiziereinheit eingeleitet und dort rektifiziert. Das Kopfprodukt dieser Kolonne III war frei von Acrylsäure. Es wurde in einem Oberflächenkondensator 16 kondensiert und in einen Abscheider 17 geführt. Dort trennte sich eine organische Phase, die 70 Gew.% Butanol, 12 Gew.-% Butylacrylat, ≤ 13 Gew.-% Wasser, 4 Gew.-% Butylacetat und 2000 ppm Dibutylether enthielt. Sie wurde vollständig durch die Leitung 18 als Rücklauf in die Kolonne III zurückgeführt. Die bei der Trennung entstandene wäßrige Phase, die noch 6 Gew.% Butanol, 300 ppm Butylacrylat, 750 ppm Butylacetat enthielt, wurde zwecks Erhöhung des Reaktionsumsatzes vollständig abgetrennt und über die Leitung 19 dem Kondensator 20 der nachfolgenden Rektifikationskolonne I zugeführt.

Der aus dem zweiten Reaktor 6 flüssig ablaufende Rohester wurde über Leitung 21 der Rektifikationskolonne I zugeführt. Er enthielt neben 0,2 Gew.-% Wasser und maximal 20 ppm Dibutylether das gewünschte Produkt n-Butylacrylat zu 78 Gew.-%, die nicht umgesetzten Edukte Butanol und Acrylsäure zu je ca. 4 Gew.-% sowie bis zu 5 Gew.-% Katalysator. Der Rest waren schwersiedende Nebenprodukte, insbesondere Oxiesterverbindungen.

Acrylsäure und Schwersieder wurden in der bei Umgebungsdruck betriebenen Rektifikationskolonne I, die mit 25 Dual-Flow-Böden mit einem Durchmesser von 50 mm ausgestattet war, mit einem Teil des Produktes und des Alkohols als Sumpfprodukt (Prod. II) abgetrennt. Das Sumpfprodukt (Prod. II) enthielt 20 Gew.-% Acrylsäure, 45 Gew.-% Butylacrylat, 3 Gew.-% Butanol, 8 Gew.-% Wasser. Eine Teilmenge von ca. 45 % der durch die Leitung 21 zugeführten Zulaufmenge wurde über die Leitung 22 in den ersten Reaktionsbereich zurückgeführt.

Der Großteil der Schwersieder (bis zu 80 % der zugeführten Menge) wurde dabei im Sumpf der Rektifikationskolonne I in Edukte und Produkte gespalten. Dabei entstanden aufgrund des hohen Acrylsäure- und Wassergehaltes des Sumpfproduktes bei einer Sumpftemperatur von 105°C nur unwesentliche Mengen an leichtsiedenden Nebenprodukten (≤ 200 ppm Dibutylether). Diese Nebenprodukte wurden mit dem Hauptproduktstrom als leichtsiedendes Minimum-Heteroazeotrop über Kopf der Kolonne I abgetrennt und über Leitung 23 dem Kondensator 20 zugeführt. Sowohl die Flüssigkeit in der Kolonne, als auch das Kopfprodukt zerfielen dabei bei einer Temperatur von 95°C in eine wäßrige und eine organische Phase. Zur Aufrechterhaltung des Heteroazeotropes in der Kolonne I wurde diese aus dem Dekanter 24 mit wäßriger Phase durch Leitung 25 und organischer Phase durch Leitung 26 als Rücklauf beaufschlagt. Die wäßrige Phase hatte dabei ≤ 3 Gew.-% organische Bestandteile, vorwiegend Butanol. Die organische Phase hatte 75 bis 85 Gew.-% Butylacrylat, 14 bis 20 Gew.-% Butanol, 2 bis 3 Gew.-% Wasser, 1500 ppm Butylacetat. Das dem Reaktionsumsatz entsprechende Überschußwasser wurde durch Leitung 27 ausgeschleust.

Auf die Zulaufmenge an Edukten in die Veresterung bezogen, wurden 5 Gew.-% des Sumpfproduktes (Prod. II) über eine Leitung 28 ausgeschleust und einem wandbeheizten Rührkessel IV zugeführt. Dort wurde das Produkt bei Umgebungsdruck und 180°C bis zum deutlichen Viskositätsanstieg diskontinuierlich eingeengt. Zunächst wurden die darin noch enthaltende Edukte Butanol und Acrylsäure neben dem Produkt Butylacrylat abdestilliert (Prod. III). Der Destillatanteil, bezogen auf die Einsatzmenge, betrug bis ca. 65 Gew.-%. Bei der anschließenden Spaltung der Schwersieder, die bis ca. 85 % Destillatanteil, bezogen auf die Einsatzmenge geführt wurde, wurden erst gegen Ende leichtsiedende Nebenprodukte wie Butene und Dibutylether in geringem Umfang gebildet. Der niedergeschlagene Brüden (Prod. IV) aus der Spaltung im Rührkessel IV bestand im wesentlichen aus Acrylsäure, Butylacrylat, Butanol und Wasser. Er wurde unmittelbar in den Sumpf der Kolonne I für die Schwersiederabtrennung zurückgeschleust. Eine weitere Rektifikation erfolgte nicht.

Das schwersieder- und acrylsäurefreie organische Kopfprodukt (Prod. I) der Azeotropdestillation in der Kolonne I wurde über die Leitung 31 einer bei einem Kopfdruck von 450 mbar betriebenen Destillationskolonne II zugeführt, die mit 25 Dual-Flow-Böden mit einem Durchmesser von 50 mm ausgestattet war, und dort rektifiziert. Butanol, Restwasser und eventuell enthaltene Leichtsieder wurden dabei als Kopfprodukt über Leitung 32 abgezogen (Prod. V). Es enthielt 65 bis 70 Gew.-% Butanol, 20 bis 30 Gew.-% Butylacrylat, 8 bis 10 Gew.-% Wasser, ≤ 500 ppm Dibutylether, ≤ 4000 ppm Butylacetat. Dieses Kopfprodukt (Prod. V) wurde in einem Kondensator 33 kondensiert und eine Teilmenge von 60 % wurde durch Leitung 34 als Rücklauf auf den Kopf der Rektifikationskolonne II zurückgeführt. Die Restmenge wurde durch Leitung 35 zusammen mit dem durch Leitung 12 zugeführten Frischalkohol der Veresterung über die erste Kolonne 1 zugeführt. Das Butylacrylat wurde im Sumpf dieser Kolonne II aufkonzentriert und zur Einhaltung der gewünschten Farbzahl sowie zur Abtrennung des Prozeßstabilisators als dampfförmiger Seitenaustrag durch Leitung 36 abgezogen, im Kondensator 37 kondensiert und durch Leitung 38 abgeführt. Das Reinprodukt enthielt ≤ 50 ppm Butanol, ≤ 50 ppm Dibutylether, ≤ 150 ppm Wasser, ≤ 50 ppm Acrylsäure.

Ein kleiner Sumpfabzugsstrom (Prod. VI) in einer Menge ≤ 2 Gew.-% des Kolonnenzulaufes wurde über eine Leitung 39 zum Sumpf der Schwersiederabtrennung in die Kolonne I geleitet.

Durch die Leitung 40 wurde aus dem Rührbehälter IV der Rückstand ausgetragen. Leitung 41 verband die Kolonnen III und II mit einer Vakuumpumpe. Durch die Leitung 42 wurde Abluft von der Kolonne 2 abgeführt. Der Sumpf der Kolonnen I und II wurde über Umlaufverdampfer 43 bzw. 44 beheizt.

Der Reinester wies eine Reinheit von ≥ 99.9 % auf, die Ausbeute bezogen auf Acrylsäure und Butanol betrug jeweils 98 % der Theorie.

In einem weiteren Versuch wurde der zweite Veresterungsreaktor 6 außer Betrieb gesetzt, und der Rohester aus dem ersten Reaktor 5 wurde über die Leitung 7 direkt in den Sumpf der Kolonne I eingeleitet. Die Umsetzung wurde bei 105°C durchgeführt. Bei gleichbleibenden Feedströmen und damit gegenüber der Variante mit zwei Reaktoren verringerter Verweilzeit konnte bei sonst identischen Verfahrensparametern ein Rohester mit 71 Gew.- des gewünschten Produktes n-Butylacrylat, 0,4 Gew.-% Wasser, maximal 20 ppm Dibutylether, einem Eduktgehalt (Butanol und Acrylsäure) von je ca. 7 Gew.-% sowie bis zu 5 Gew.-% Katalysator erhalten werden. Der Rest waren schwersiedende Nebenprodukte, insbesondere Oxyesterverbindungen.

Der so erzeugte Rohester wurde analog zum ersten Versuch bei identischen Verfahrensparametern im Aufarbeitungsteil zu 99,9%-igem Reinprodukt, bei einer auf die Edukte bezogenen Gesamtausbeute von 98 %, aufgereinigt.

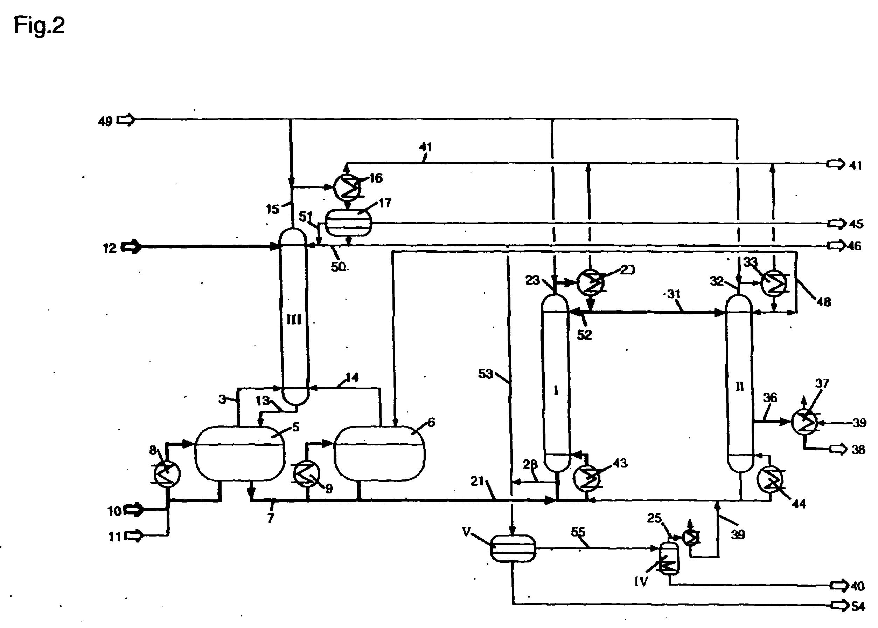

Mit der in Fig. 2 dargestellten Anlage zur Durchführung des erfindungsgemäßen Verfahrens wurde 2-Ehtlyhexylacrylat hergestellt.

Die Veresterungsreaktion durch Umsetzung von 2-Ethylhexanol und Acrylsäure erfolgte in einer zweistufigen Veresterungskaskade, die aus zwei gleichvolumigen Reaktoren 5 und 6 bestand. Dem Reaktor 5 wurden über eine Leitung 10 Acrylsäure und über eine Leitung 11 p-Toluolsulfonsäure als Katalysator in einem Umlaufverdampfer 8 zugeführt. Die Reaktionskomponente 2-Ethylhexanol wurde über eine Leitung 12 auf den Kopf einer Destillationskolonne III aufgegeben, deren unteres Ende über eine Leitung 13 mit dem Reaktor 5 verbunden war. Der bei der Veresterung in den Reaktoren 5 und 6 gebildete, das Reaktionswasser enthaltende Dampf wurde über Leitungen 3 und 14 der Destillationskolonne III zugeführt, die zehn theoretische Böden aufwies und bei einem Druck von 270 mbar betrieben wurde. Dieser Druck wurde durch eine Leitung 15, die zu einem Kühler 16 führte, über eine zu einer Vakuumpumpe führenden Leitung 41 aufrechterhalten. Das im Kühler 16 gebildete Kondensat wurde im Abscheider 17 in zwei flüssige Phasen aufgetrennt. Abgetrennte Oktene wurden durch Leitung 45 abgeführt. Reaktionswasser wurde durch die Leitung 46 abgeführt.

Da die Edukte dem Reaktor 5 mit Umgebungstemperatur zugeführt wurden und bei der Reaktion eine hohe Wassermenge gebildet wird, ist für die Aufrechterhaltung einer Reaktionstemperatur von 110 °C ein hoher Wärmeeintrag in den ersten Reaktor 5 erforderlich. Dies macht den Einsatz eines außenliegenden Umlaufverdampfers 8 möglich, der auch im Hinblick auf die notwendige ausreichende Durchmischung des Kesselinhalts besonders vorteilhaft ist. Das aus dem Reaktor 5 über Leitung 7 ausgetragene Sumpfprodukt wurde über einen weiteren Umlaufverdampfer 9 dem zweiten Reaktor 6 zugeführt, in dem eine Temperatur von 120 °C eingestellt wurde. Auch der zweite Verdampfer 9 wurde als Umlaufverdampfer ausgeführt, um trotz wesentlich geringeren Wärmeeintrags eine für die Durchmischung des Kesselinhalts ausreichende Umlaufmenge sicherzustellen. Auch hier wurde Magerluft als Co-Stabilisator zugegeben. Der abnehmenden Acrylsäure- und Wasser-Konzentration angepaßt, wurde der zweite Reaktor 6 bei erhöhter Temperatur betrieben. Das im Reaktor 6 entstehende Sumpfprodukt wurde durch Leitung 21 ausgetragen. Der gesamte Reaktionsaustrag, der das Zielprodukt, d.h. das gebildete 2-Ethylhexylacrylat sowie alle leichter siedenden Edukte und Nebenprodukte enthält, wurde über Leitung 21 und einen weiteren Umlaufverdampfer 43 in den unteren Teil einer Rektifikationskolonne I eingeleitet, die als eine Verstärkungskolonne ausgebildet war und mit zehn Dual-Flow-Böden der Schwersiederabtrennung diente. Das Zielprodukt, nämlich 2-Ethylhexylacrylat, sowie alle leichtersiedenden Edukte und Nebenprodukte (Prod. VII) wurde über Kopf durch Leitung 23 abgeführt und nach Durchströmen eines mit der Vakuumleitung 41 verbundenen Kühlers über Leitung 31 dem Kopf einer als eine Abtriebssäule betriebenen Reinkolonne II zugeführt.

Die Kolonne I für die Schwersiederabtrennung wurde bei einem Sumpfdruck von 100 mbar und einem Kopfdruck von 70 mbar betrieben. Die Temperatur betrug 150 °C.

Der in der Kolonne I für die Schwersiederabtrennung anfallende Sumpf (Prod. VIII) wurde über eine Leitung 28 ausgetragen, auf 50°C abgekühlt und einer Extraktionseinheit zugeführt. Unter Zugabe eines Teiles des Veresterungswassers durch Leitung 53 wurde der Gehalt an p-Toluolsulfonsäure in der organischen Phase auf für die Spaltung optimale 1,5 % abgesenkt. Der mit bis zu 30 % p-Toluolsulfonsäure beladene, ablaufende Wasserstrom wurde durch Leitung 54 abgezogen, die organische Phase durch Leitung 55 einer Destillationseinheit IV zugeführt. In dieser wurde bei einer Temperatur von 180 °C und einem Druck von 60 mbar diskontinuierlich zunächst noch enthaltenes Produkt abgedampft. Anschließend wurde der hoch p-Toluolsulfonsäure und Oxyester enthaltende Rückstand in Edukte, Zielprodukt, Wasser und die als Nebenprodukt anfallenden Oktene gespalten. Das vereinigte Kopfprodukt aus der Spaltung wurde über Leitung 25 abgezogen, verflüssigt und über Leitung 39 in den Sumpf der Schwersiederabtrennung zurückgeführt. Der verbleibende zähflüssige Rückstand wurde durch Leitung 40 abgezogen und in einer Rückstandsverbrennung entsorgt.

Aus dem der Reinkolonne II durch Leitung 31 über Kopf zugeführten Produkt (Prod. VII) wurden die noch vorhandenen Edukte und leichtersiedenden Nebenkomponenten über Leitung 32 abgezogen (Prod. XII) und einem Kondensator 33 zugeführt. Das dabei anfallende Kondensat wurde über Leitung 48 in die zweite Stufe 6 der Veresterungskaskade zurückgeführt. Der Sumpf der Kolonne II wurde durch einen, den in der Veresterung eingesetzten Apparaten analogen Umlaufverdampfer 44 beheizt.

Das Reinprodukt 2-Ethylhexylacrylat wurde über Leitung 36 dampfförmig abgezogen und über einen berieselten Demister dem Reinproduktkondensator 37 zugeführt, um Farbzahlprobleme zu vermeiden und das Umstabilisieren des Produkts von Phenothiazin als Prozeßinhibitor auf Hydrochinonmonomethylether als Lagerstabilisator zu ermöglichen. Das Reinprodukt wurde durch Leitung 38 abgeführt. Der Lagerstabilisator wurde durch Leitung 39 zugeführt. Das als Prozeßinhibitor eingesetzte Phenothiazin wurde über Leitung 49 den Rektifikationskolonnen I, II, III über Kopf zugeführt.

Im folgenden soll nun ein spezielles Ausführungsbeispiel beschrieben werden, das mit einer Versuchsapparatur durchgeführt worden ist, wie sie in der Figur 2 dargestellt ist. Dabei wurden zwei Veresterungskessel 5 und 6 mit je 2 l Nutzvolumen eingesetzt, auf die eine Glasbodenkolonne mit einem Durchmesser von 50 mm aufgesetzt war, die mit 20 Glockenböden sowie einem Phasenscheider am Kolonnenkopf ausgestattet war. Der Betriebsdruck betrug 270 mbar. Zur Beheizung der Veresterungskessel wurden Umlaufverdampfer eingesetzt. Bei einer Verweilzeit von 4 Stunden wurde Acrylsäure mit 2-Ethylhexanol im stöchiometrischen Verhaltnis unter Zugabe von 1,5 Gew.-% wäßriger p-Toluolsulfonsäure unter kontinuierlicher und destillativer Abtrennung des gebildeten Reaktionswassers zu 2-Ethylhexylacrylat (EHA) umgesetzt. Die Temperatur im ersten Veresterungskessel 5 betrug 110 °C, im zweiten Veresterungskessel 6 betrug die Temperatur 120 °C. Im Austrag des ersten Reaktors 5 wurde eine Konzentration an EHA von 70 Gew.-% und im Austrag des zweiten Reaktors 6 von 82 Gew.-% erreicht. Leichtsiedende Nebenkomponenten (in der Hauptsache in der Spaltung gebildete Oktene) wurden im Kopf der Veresterungskolonne III soweit konzentriert, daß der über Leitung 45 abgezogene Ausschleusstrom nur noch < 10% Wertkomponenten, d.h. 2-Ethylhexanol und 2-Ethylhexylacrylat, enthielt. Durch wäßrigen Kolonnenrücklauf wurden über die gesamte Kolonnenhöhe zwei flüssige Phasen eingestellt. Dadurch konnte die Acrylsäure im Kolonnenkopf auf < 100 ppm abgereichert werden. Das in stöchiometrischer Menge anfallende Veresterungswasser war zu etwa 1,5% mit organischen Verbindungen (in der Hauptsache 2-Ethylhexanol und Oktenen) im Gleichgewicht beladen.

Der Veresterungsaustrag durch Leitung 21 wurde in einer mit Umlaufverdampfer und stehendem Rohrbündelkondensator ausgestatteten Laborkolonne I mit 50 mm Durchmesser und 10 Dual-Flow-Böden von der Katalysatorsäure und den gebildeten Schwersiedern befreit. 5% der zulaufenden Rohestermenge wurden bei einem Rücklaufverhältnis von 0,5 aus dem Sumpf der Kolonne als Schwersiederaustrag in die Spaltung abgezogen, der Rest als schwersiederfreies (Oxiester < 10 ppm) Kopfprodukt. Der Zulauf erfolgte unmittelbar in den Kolonnensumpf und die Kolonne I wurde als reine Verstärkungssäule betrieben. Bei einem Kopfdruck von 80 mbar konnte eine maximale Sumpftemperatur von 150 °C eingehalten werden. Das schwersiederfreie Kopfprodukt wurde in einer mit 25 Dual-Flow-Böden ausgestatteten Laborkolonne II von 50 mm Durchmesser bei einem Kopfdruck von 80 mbar und einer maximalen Sumpftemperatur von 140 °C in eine die Edukte Acrylsäure und 2-Ethylhexanol sowie 50 Gew.-% 2-Ethylhexylacrylat enthaltende Kopffraktion und das Reinprodukt aufgetrennt. Die Kopffraktion wurde in den zweiten Veresterungsreaktor 6 zurückgeführt. Das Reinprodukt wurde frei von Schwersiedern und Prozeßstabilisatoren dampfförmig aus dem Kolonnensumpf, der mit einem Naturumlaufverdampfer beheizt wurde, abgezogen und in einem durch Inertenüberdeckung geregelten Kondensator verflüssigt. Dabei wurde ein Gehalt von > 99,8 Gew.-% 2-Ethylhexylacrylat erreicht. Eine Aufpegelung schwersiedender Spurenkomponenten im Kolonnensumpf wurde durch einen flüssigen Sumpfabzug in den Sumpf der Schwersiederabtrennung von 2% der zulaufenden Flüssigkeitsmenge verhindert. Der Sumpfaustrag der Schwersiederabtrennung wurde nach teilweiser Extraktion der Katalysatorsäure mit Wasser in einem diskontinuierlich betriebenen Spaltkessel IV bei 60 mbar und einer Maximaltemperatur von 180 °C auf 20% seiner ursprünglichen Masse eingeengt. Der entstehende Rückstand enthielt neben der Katalysatorsäure p-Toluolsulfonsäure nicht spalt- und verdampfbare Schwersieder in hoher Konzentration. Dieser Rückstand war nicht weiter im Prozeß verwertbar und wurde abgezogen. Das zu 80% aus EHA, 10 bis 12% Oktenen sowie aus Acrylsäure, Wasser und 2-Ethylhexanol bestehende Kopfprodukt wurde in einem Wärmeübertrager niedergeschlagen und in den Sumpf der Schwersiederabtrennung rückgeführt.

Im kontinuierlichen, stationären Betrieb dieser Versuchsanlage konnte eine Eduktausbeute von 98% erreicht werden. Nur 2% der eingesetzten Edukte gingen als Nebenprodukte verloren.

Als Stabilisatorlösung diente eine 2%ige Phenothiazinlösung in 2-Ethylhexanol, die jeweils in die Kopfkondensatoren der einzelnen Verfahrensstufen in einer Aufwandmenge von 100 ppm, bezogen auf den jeweiligen Zulaufstrom der Stufe, entsprechend dosiert wurde. Alle Naturumlaufverdampfer wurden mit Luft als Co-Stabilisator beaufschlagt.

Besonders vorteihaft bei dem vorstehend beschriebenen Verfahren ist die Abtrennung aller schwersiedenden Neberkomponenten und insbesondere des Katalysators aus dem Veresterungsaustrag in Kolonne I. Dadurch wird eine Rückspaltung der Schwersieder und/oder des Zielproduktes im Sumpf der Reinkolonne II in Edukte und damit eine Verunreinigung des Reinproduktes mit leichtsiedenden Spaltprodukten und insbesondere Acrylsäure sicher vermieden.

Werden, wie bei konventionellen Verfahrensweisen üblich, aus dem Veresterungsaustrag zunächst die leichter als der Zielester siedenden Komponenten (insbesondere Acrylsäure und Ausgangsalkanol) abgetrennt, so ist es aufgrund der dann im Sumpf der Reinkolonne in Anwesenheit von Katalysator und Schwersiedern einsetzenden Spaltreaktionen nicht möglich, leichtsieder- und insbesondere acrylsäurefreies Reinprodukt zu gewinnen, wie durch das nachfolgend beschriebene Ausführungsbeispiel nachgewiesen wurde:

Aus Leitung 21 entnommener Veresterungsaustrag wurde zunächst in Kolonne I von allen leichter als der Zielester siedenden Nebenkomponenten sowie den Edukten Acrylsäure und 2-Ethylhexanol befreit. Aus dem Sumpf der Kolonne I wurde dann der leichtsiederfreie, aber mit Schwersiedern und insbesondere dem Katalysator verunreinigte Rohester entnommen und in einer mit 25 Dual-Flow-Böden ausgestatteten, 50 mm durchmessenden Laborkolonne bei einem Rücklaufverhältnis von 2 rektifiziert. Der dabei im Kopf der Kolonne entnommene Rohester war mit 1400 ppm Acrylsäure verunreinigt, obwohl der zulaufende Rohester acrylsäurefrei war; d.h. die im Zielester nachgewiesene Acrylsäure kann nur durch Spaltreaktionen im Sumpf der Kolonne entstanden sein. Eine destillative Abtrennung der Acrylsäure ist jedoch bei der Gewinnung des Reinesters als Kopfprodukt nicht möglich, da Acrylsäure gegenüber dem Zielester einen Leichtsieder darstellt.

Den vorstehend beschriebenen Versuchsergebnissen sowie weiteren Untersuchungen konnte entnommen werden, daß es zweckmäßig ist, die beiden Veresterungsreaktoren 5 und 6 bei Drücken zwischen 180 bis 500 mbar, vorzugsweise 180 bis 350 mbar, zu betreiben. Dabei kann die Temperatur im ersten Reaktor 5 bei 80 bis 120 °C, im zweiten Reaktor 6 bei 100 bis 140 °C liegen. Als Katalysator für die Veresterungsreaktion in den Reaktoren 5 und 6 haben sich saure Katalysatoren, insbesondere organische Sulfonsäuren und hier besonders p-Toluolsulfonsäure in einer Menge von 0,1 bis 4, vorzugsweise 0,5 bis 2 Gew.-%, als besonders vorteilhaft erwiesen.

Die Verweilzeit von (Meth)acrylsäure und Alkanol in den Reaktoren liegt zwischen 0,5 und 8 Stunden, vorzugsweise zwischen 1 und 6 Stunden. Von der durch Leitung 21 der Rektifikationskolonne I zugeführten Rohestermenge kann bei einem Rücklaufverhältnis von 0,5 eine Menge < 10, vorzugsweise < 5 Gew.-%, aus dem Sumpf der Kolonne als Schwersiederaustrag in die Spaltung abgezogen werden. Der Kopfdruck der Kolonne I kann 50 bis 400 mbar, vorzugsweise < 120 mbar, betragen. Die maximale Sumpftemperatur dieser Kolonne beträgt vorzugsweise ≤ 150 °C. Das schwersiederfreie Kopfprodukt der Kolonne I, das über die Leitung 26 der Kolonne II zugeführt wurde, kann dort bei einem Kopfdruck von 50 bis 400 mbar, vorzugsweise bei einem Druck < 120 mbar, und bei einer Sumpftemperatur < 140 °C bearbeitet werden. Bei optimaler Einstellung der vorgenannten Werte kann durch Leitung 36 ein Produkt abgezogen werden, dessen Gehalt an Reinprodukt, im Falle des Ausführungsbeispiels an 2-Ethylhexylacrylat, > 99,8 Gew.-% beträgt.

## Patentansprüche

1. Verfahren zur kontinuierlichen Herstellung von Alkylestern der (Meth)acrylsäure durch Umsetzung von (Meth)acrylsäure mit 1 bis 8 C-Atome aufweisenden einwertigen Alkanolen in Gegenwart eines sauren Veresterungkatalysators in einer Reaktionszone, bei dem aus der Reaktionszone ein Produktgemisch ausgetragen wird, das den gebildeten Alkylester der (Meth)acrylsäure, den Katalysator und die im Verlauf der Veresterung gebildeten höher als der Alkylester der (Meth)acrylsäure siedenden Nebenprodukte enthält und aus dem der Alkylester der (Meth)acrylsäure in einer Trennzone destillativ abgetrennt wird, dadurch gekennzeichnet, daß man das aus der Reaktionszone ausgetragene Produktgemisch einer Rektifikationseinheit (I) zuführt, in dieser das ausgetragene Produktgemisch rektifikativ in wenigstens ein den Alkylester der (Meth)acrylsäure enthaltendes Produkt (I) und ein den Katalysator enthaltendes Produkt (II) auftrennt, das Produkt (I) einer weiteren Rektifikationseinheit (II) zuführt und in dieser den Alkylester der (Meth)acrylsäure rektifikativ abtrennt.

2. Verfahren nach Anspruch 1, dadurch gekennzeichnet, daß die Veresterung in der Reaktionszone in homogener flüssiger, an Lösungsmittel freier Phase bei erhöhter Temperatur durch Umsetzung von (Meth)acrylsäure und den Alkanolen, insbesondere mit 4 bis 8 C-Atomen, in einem Molverhältnis von 1:0,75 bis 1:2, vorzugsweise 1:0,9 bis 1:1,1, ganz besonders 1:1, so erfolgt, daß man der Reaktionszone die (Meth)acrylsäure, das Alkanol und den Katalysator zuführt, während der Verweilzeit das gebildete Wasser als Bestandteil eines Alkanol umfassenden Gemisches über Kopf einer der Reaktionszone aufgesetzten Rektifikationseinheit (III) rektifikativ abtrennt, das dabei anfallende Destillat in eine Alkanol enthaltende organische und in eine Wasser enthaltende wäßrige Phase auftrennt, organische Phase und gegebenenfalls wäßrige Phase in die Rektifikationseinheit (III) zurückführt, das Produktgemisch aus der Reaktionszone austrägt und einer Trennzone zuführt, wobei die Reaktionszone insbesondere aus einer Kaskade von mindestens zwei hintereinander geschalteten Reaktionsbereichen besteht, und der Austragsstrom eines Reaktionsbereichs einen Zulaufstrom eines nachfolgenden Reaktionsbereichs bildet, und die Kaskade, insbesondere 2 bis 4 voneinander räumlich getrennte Reaktionsbereiche aufweist.

3. Verfahren nach Anspruch 1 oder 2, dadurch gekennzeichnet, daß die aufsteigenden Dämpfe aus den Reaktionsbereichen einer einzelnen Rektifikationseinheit zugeführt werden, deren flüssiger Rücklauf nur in den ersten Reaktionsbereich zurückgeführt wird.

4. Verfahren nach Anspruch 2 oder 3, dadurch gekennzeichnet, daß die Temperatur im ersten Reaktionsbereich 70 bis 150°C, vorzugsweise 80 bis 130°C beträgt, und im letzten Bereich 100 bis 160°C, vorzugsweise 110 bis 130°C beträgt, wobei sie insbesondere längs der Kaskade ansteigt.

5. Verfahren nach einem der Ansprüche 2 bis 4, dadurch gekennzeichnet, daß der Druck in allen Reaktionsbereichen von 100 mbar bis Atmosphärendruck, vorzugsweise 200 mbar bis 700 mbar beträgt, insbesondere in allen Reaktionsbereichen gleich ist.

6. Verfahren nach einem der Ansprüche 1 bis 5, dadurch gekennzeichnet, daß als Katalysator p-Toluolsulfonsäure und/oder eine andere organische Sulfonsäure wie Methansulfonsäure, Benzolsulfonsäure, Dodecylbenzolsulfonsäure und/oder Schwefelsäure eingesetzt werden, wobei der Gehalt an katalytisch wirksamer Säure in der Reaktionszone bezogen auf das darin enthaltene Reaktionsgemisch 0,1 bis 10 Gew.-%, vorzugsweise 0,1 bis 6 Gew.-%, an para-Toluolsulfonsäure oder einer dazu äquimolaren Menge an einer anderen organischen Sulfonsäure und/oder Schwefelsäure beträgt.

7. Verfahren nach einem der Ansprüche 1 bis 6, dadurch gekennzeichnet, daß sowohl die (Meth)acrylsäure als auch der Katalysator der Reaktionszone direkt zugeführt wird, das zu veresternde Alkanol, insbesondere n-Butanol, der Reaktionszone über die Rektifikationseinheit (III) zugeführt wird, die Rektifikationseinheit (III) aus einer Rektifikationskolonne besteht, und die Reaktionsbereiche aus Reaktoren mit Umlaufverdampfern bestehen.

8. Verfahren nach Anspruch 7, dadurch gekennzeichnet, daß die am Kopf der Rektifikationseinheit (III) anfallende wäßrige Phase im wesentlichen vollständig ausgeschleust wird, die anfallende organische Phase im wesentlichen vollständig in die Rektifikationseinheit (III) zurückgeführt wird, das aus der Reaktionszone ausgetragene Produktgemisch der Rektifikationseinheit (I) unter Zusatz von Wasser zugeführt wird, wobei das unter Zusatz von Wasser in die Rektifikationseinheit (I) geführte Produktgemisch in dieser in ein den Katalysator und die verbliebene (Meth)acrylsäure enthaltendes Produkt (II) und in ein den n-Butylester der (Meth)acrylsäure, verbliebenes n-Butanol und Wasser enthaltendes Produkt (I) aufgetrennt wird, das dann weiterhin in eine den n-Butylester der (Meth)acrylsäure und n-Butanol enthaltende organische Phase und in eine wäßrige Phase aufgetrennt wird.

9. Verfahren nach Anspruch 8, dadurch gekennzeichnet, daß wäßrige Phase in die Rektifikationseinheit (I) zurückgeführt wird, in der Rektifikationseinheit (I) eine flüssige wäßrige und eine flüssige organische Phase vorhanden ist, die Rektifikationseinheit (I) eine Rektifikationskolonne (I) ist, das aus der Reaktionszone ausgetragene Produktgemisch dem unteren Teil der Rektifikationskolonne (I) zugeführt wird und der Wasserzusatz im oberen Teil der Rektifikationskolonne (I) erfolgt, und ein Teil der den n-Butylester der (Meth)acrylsäure und n-Butanol enthaltenden anfallenden organischen Phase in den oberen Teil der Rektifikationskolonne (I) zurückgeführt wird.

10. Verfahren nach Anspruch 8 oder 9, dadurch gekennzeichnet, daß das in der Rektifikationseinheit (I) anfallende, den Katalysator und die verbliebene (Meth)acrylsäure enthaltende Produkt (II) im wesentlichen vollständig in die Reaktionszone, vorzugsweise in den ersten Reaktionsbereich, entweder direkt und/oder über die Rektifikationseinheit (III), zurückgeführt wird, mindestens ein Teil des in der Rektifikationseinheit (I) anfallenden Produktes (II) ausgetragen und einer Destillationseinheit (IV) zugeführt und in dieser in ein n-Butanol, (Meth)acrylsäure und n-Butylester der (Meth)acrylsäure enthaltendes Produkt (III) und ein den Katalysator sowie höher als der n-Butylester der (Meth)acrylsäure siedende Komponenten enthaltendes Produkt (IV) aufgetrennt wird, das Produkt (III) in die Rektifikationseinheit (I) und/oder in die Reaktionszone zurückgeführt wird, die organische Phase des Produkts (I) einer Rektifikationseinheit (II) zugeführt und in dieser aufgetrennt wird in
a) ein verbliebenes n-Butanol und leichter als n-Butyl(meth)acrylat siedende Komponenten enthaltendes Produkt (V),
b) n-Butyl(meth)acrylat und
c) ein schwerer als n-Butyl(meth)acrylat siedendes Produkt (VI),
das Produkt (V) in die Reaktionszone zurückgeführt wird, vorzugsweise über die Rektifikationseinheit (III), das Produkt (VI) in die Rektifikationseinheit (I) zurückgeführt wird, die Rektifikationseinheit (II) eine Rektifikationskolonne (II) ist und das Produkt (V) im oberen Teil der Rektifikationskolonne (II), das Produkt (VI) aus dem Sumpf der Rektifikationskolonne (II) und das n-Butyl(meth)acrylat als dampfförmiger Seitenabzug im unteren Teil der Rektifikationskolonne (II) abgetrennt wird.

11. Verfahren nach einem der Ansprüche 1 bis 7, dadurch gekennzeichnet, daß das verwendete Alkanol 2-Ethylhexanol ist, wobei von der am Kopf der Rektifikationseinheit (III) anfallenden wäßrigen Phase ein Teil in die Rektifikationseinheit (III) zurückgeführt wird, das aus der Reaktionszone ausgetragene Produktgemisch der Rektifikationseinheit (I) zugeführt wird, das in die Rektifikationseinheit (I) geführte Produktgemisch in der Rektifikationseinheit (I) in ein den 2-Ethythexylester der (Meth)acrylsäure, verbliebenes 2-Ethylhexanol und verbliebene (Meth)acrylsäure enthaltendes Produkt (VII) und in ein den Katalysator und schwerer als der 2-Ethylhexylester der (Meth)acrylsäure siedende Komponenten enthaltendes Produkt (VIII) aufgetrennt wird, das aus der Reaktionszone ausgetragene Produktgemisch dem unteren Teil der Rektifikationskolonne (I) zugeführt wird, und das Produkt (VIII) aus dem Sumpf der Rektifikationskolonne (I) und das Produkt (VII) am Kopf der Rektifikationskolonne (I) gewonnen wird.

12. Verfahren nach Anspruch 11, dadurch gekennzeichnet, daß ein Teil des Produktes (VIII) in die Reaktionszone, vorzugsweise in den ersten Reaktionsbereich, entweder direkt und/oder über die Rektifikationseinheit (III) zurückgeführt wird, ein Teil des Produktes (VIII) ausgetragen und einer Destillationseinheit (IV) zugeführt und in dieser in ein 2-Ethylhexanol, (Meth)acrylsäure und 2-Ethylhexylester der (Meth)acrylsäure enthaltendes Produkt (IX) und ein den sauren Veresterungskatalysator sowie höher als der 2-Ethylhexylester der (Meth)acrylsäure siedende Komponenten enthaltendes Produkt (X) aufgetrennt wird, das Produkt (IX) in die Rektifikationseinheit (I) und/oder in die Reaktionszone zurückgeführt wird, aus dem Produkt (VIII) und/oder dem Produkt (X) durch Extraktion mit Wasser Katalysator abgetrennt und die anfallende wäßrige Phase in die Reaktionszone zurückgeführt wird und zur Extraktion ein Teil der in der Rektifikationseinheit (III) anfallenden wäßrigen Phase verwendet wird.

13. Verfahren nach Anspruch 11 oder 12, dadurch gekennzeichnet, daß das der Rektifikationseinheit (I) entnommene Produkt (VII) einer Rektifikationseinheit (II) zugeführt und in dieser aufgetrennt wird in
a) ein verbliebenes 2-Ethylhexanol und leichter als 2-Ethylhexyl(meth)acrylat siedende Komponenten enthaltendes Produkt (XI),
b) 2-Ethylhexyl(meth)acrylat und
c) ein schwerer als 2-Ethylhexyl(meth)acrylat siedendes Produkt (XII),
wobei das Produkt (XI) in die Reaktionszone zurückgeführt wird, vorzugsweise über die Rektifikationseinheit (III), das Produkt (XII) in die Rektifikationseinheit (I) zurückgeführt wird, das Produkt (XI) im oberen Teil der Rektifikationskolonne (II), das Produkt (XII) aus dem Sumpf der Rektifikationskolonne (II) und das 2-Ethylhexyl(meth)acrylat als dampfförmiger Seitenabzug im unteren Teil der Rektifikationskolonne (II) abgetrennt wird und ein Teil der in der Rektifikationseinheit (III) anfallenden organischen Phase zur Ausschleusung leichtsiedender Nebenprodukte ausgetragen wird.
